# EUROPEAN PATENT APPLICATION

(11) **EP 1 260 241 A1**
(43) Date of publication of application: **27.11.2002**
(21) Application number: 01908192.6
(22) Date of filing: 01.03.2001
(51) Int. Cl.: A61M 5/178, A61M 5/28

(54) **PREFILLED SYRINGE ASSEMBLY**

(30) Priority: 02.03.2000 JP 2000056907
(71) Applicant: Daikyo Seiko, LTD., Tokyo 131-0031 (JP); CHUGAI SEIYAKU KABUSHIKI KAISHA, Tokyo, 115-8543 (JP); Sugihara, Miyo, Saitama 336-0932 (JP)
(72) Inventor: SUGIHARA, Shigeru DI, DECEASED (JP); SUDO, Morihiro, c/o Daikyo Seiko Ltd., Tokyo 131-0031 (JP); WATANABE, Yoshirou, c/o Chugai Seiyaku K. K., Tokyo 115-8543 (JP)
(74) Representative: Schaumburg, Thoenes & Thurn
(86) International application number: JP0101561
(87) International publication number: WO01064266

(57) **Abstract**

A pre-filled syringe assembly is achieved which prevents, with a strong force, a stopper provided therein from being pulled out, and wherein the stopper can be prevented from being scratched.

The pre-filled syringe assembly includes a barrel and a flanged fixing member which is screwed to the barrel, wherein a stopper-pull-out prevention portion is provided by the flanged fixing member. It is desirable for a reuse-prevention projection and a notch to be provided on one and the other of the flanged fixing member and the plunger, which engage with each other when the plunger is inserted into the barrel up to a position therein whereby a liquid drug in the barrel is totally discharged. An air evacuation groove can be provided on the inner peripheral surface of the barrel, which is defined by a gap between the barrel and the stopper when the stopper is positioned at the open end side of the barrel.

## Description

### FIELD OF THE INVENTION

The present invention relates to a single dose, pre-filled syringe assembly which contains a predetermined dose of a pre-chosen liquid drug.

### BACKGROUND OF THE INVENTION

Pre-filled syringe assemblies are advantageous for emergency use because a liquid drug (liquid medicine) does not have to be filled before use, and are superior to conventional reusable syringes because there is little chance of the syringe assemblies being contaminated with germs.

Pre-filled syringe assemblies are generally required to have a function of preventing the stopper from being pulled out of the cylindrical barrel when a pressure is applied to the pre-filled liquid drug, and also a reuse prevention function. The stopper-pull-out prevention function prevents a pressurized pre-filled liquid drug together with the stopper from coming out of the barrel when the pre-filled liquid is injected into a high-pressurized system, e.g., when the pre-filled liquid drug is injected into a dialyzer for use in artificial dialysis or when a high-viscosity liquid drug is dispensed using an infusion pump. The reuse prevention function prevents a used syringe assembly from being re-filled with a liquid drug so as not to be reused.

Conventional pre-filled syringe assemblies with such functions have the following problems: the structure is complicated, a sufficient strength for preventing the stopper from being pulled out of the barrel cannot be achieved, and a protrusion formed on the barrel for preventing the stopper from being pulled out of the barrel or for the prevention of reuse of the syringe assembly may become a cause of scratching the stopper. It is desirable that the stopper of pre-filled syringe assemblies be of a type coated with a film which is inert with respect to the pre-filled liquid drug since the stopper is in contact with the pre-filled liquid drug for a long period of time. However, if such a film coating of the stopper is scratched, the film coating looses its original effect.

An object of the present invention is to obtain a pre-filled syringe assembly having the capability of preventing the stopper from being pulled out of the barrel with a sufficient strength, wherein the structure of the syringe assembly is simple while there is no possibility of scratching the stopper when it is inserted into the barrel. Another object of the present invention is to obtain a pre-filled syringe assembly with a simple structure preventing the syringe assembly from being reused.

### DISCLOSURE OF THE INVENTION

A pre-filled syringe assembly according to the present invention includes a cylindrical barrel having one of a cap and an injection needle provided on one end of the cylindrical barrel, the other end of the cylindrical barrel being formed as an open end; a flanged fixing member having a cylindrical portion and a finger flange, the cylindrical portion being formed to correspond to the open end of the cylindrical barrel, and the finger flange extending radially outwards from the cylindrical portion; two threaded portions formed on the cylindrical barrel around the open end thereof and the cylindrical portion of the flanged fixing member, respectively, to fix the flanged fixing member to the cylindrical barrel; and a stopper-pull-out prevention portion formed on the flanged fixing member to have a diameter smaller than each of an inside diameter of the cylindrical barrel and an outside diameter of a stopper fitted into the cylindrical barrel.

Furthermore, it is desirable for the pre-filled syringe to further include a plunger screwed into a core of the stopper; a reuse-prevention projection formed on one of the plunger and the flanged fixing member; and a non-pull-out recess formed on the other of the plunger and the flanged fixing member, the reuse-prevention projection being engaged in the non-pull-out recess when the plunger is inserted into the barrel up to a position therein whereby a liquid drug pre-filled in the barrel is totally discharged.

It is desirable for the barrel to be provided, on an inner peripheral surface thereof on the open end side of the barrel, with an air evacuation groove which forms a gap between the barrel and the stopper when the stopper is positioned at the open end side of the barrel.

The two thread portions, which are formed on the cylindrical barrel around the open end thereof and the cylindrical portion of the flanged fixing member, can include a female thread portion and a male thread portion, respectively, or vice versa.

In the embodiment wherein the cylindrical barrel and the cylindrical portion of the flanged fixing member include a female thread portion and a male thread portion, respectively, the stopper-pull-out prevention portion of the flanged fixing member can be formed by an inner end of the cylindrical portion. Furthermore, in this embodiment, the reuse-prevention projection or the non-pull-out recess which is formed on the flanged fixing member can be formed on an inner peripheral surface of the cylindrical portion of the flanged fixing member.

In the embodiment wherein the cylindrical barrel and the cylindrical portion of the flanged fixing member include a male thread portion and a female thread portion, respectively, the stopper-pull-out prevention portion of the flanged fixing member can be formed by an inwardly extending flange formed at substantially the same axial position as that of the finger flange of the flanged fixing member. Furthermore, in this embodiment, the stopper-pull-out prevention portion and the non-pull-out recess of the flanged fixing member can be formed by an inwardly extending flange formed at substantially the same axial position as that of the finger flange of the flanged fixing member.

The barrel can be made of a cycloolefin resin, and it is desirable for the stopper to be coated with a film which is inert with respect to a liquid drug which is pre-filled in the barrel, the film covering at least a portion of the stopper which is in contact with at least one of the pre-filled liquid drug and the barrel.

Although several embodiments of pre-filled syringe assemblies according to the present invention will be described below, the present invention is not limited solely to these embodiments.

### BRIEF DESCRIPTION OF THE DRAWINGS

Figure 1 shows the first embodiment of a pre-filled syringe assembly in cross section according to the present invention, showing procedures for filling a liquid drug and for assembling components of the syringe assembly;
Figure 2 is a plan view of the cylindrical barrel shown in Figure 1, viewed from the open end side of the cylindrical syringe;
Figure 3 is a perspective view of the cylindrical barrel shown in Figure 1, showing a state where the cylindrical barrel is in a process of being transferred;
Figure 4 shows the barrel, which has been assembled in a manner shown in Figure 1, and the stopper in cross section, showing a state before the barrel and the plunger are put together and a state after the barrel and the plunger are put together;
Figure 5 is a cross sectional view of the pre-filled syringe assembly shown in Figure 4, showing a state where the stopper is prevented from being pulled out of the barrel;
Figure 6 is a cross sectional view of the pre-filled syringe assembly shown in Figure 4, showing a state where the plunger is prevented from being reused;
Figure 7 is a cross sectional view of another embodiment of the pre-filled syringe assembly according to the present invention; and
Figure 8 is a cross sectional view of another embodiment of the pre-filled syringe assembly according to the present invention.

### DESCRIPTION OF THE PREFERRED EMBODIMENTS

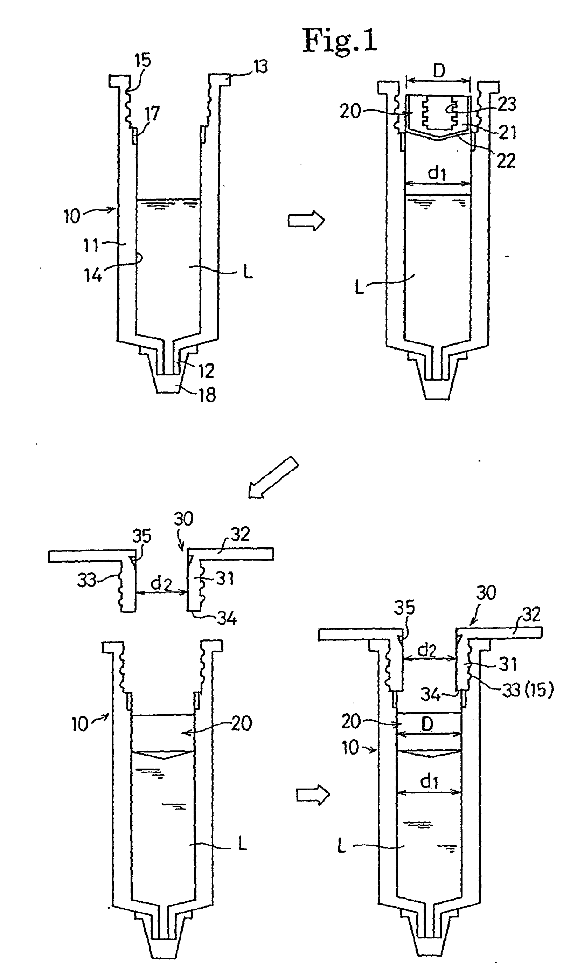

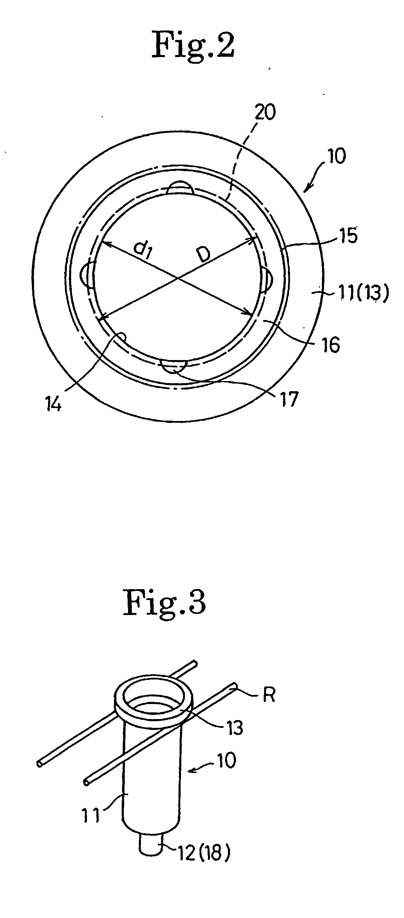

Figures 1 through 5 show the first embodiment of a pre-filled syringe assembly according to the present invention. Figure 1 shows the procedure of filling a liquid drug (liquid medicine) L into a barrel 10, fitting a stopper 20 into the barrel 10, and screwing a flanged fixing member 30 into the barrel 10 to fix the flanged fixing member 30 to the barrel 10. The barrel 10 is made of synthetic resin, preferably a cycloolefin resin. The barrel 10 has a generally cylindrical shape, and is provided at the tip thereof with a nozzle portion 12 having a small diameter. The barrel 10 is provided at the rear open end thereof with an outer flange portion 13. As shown in Figure 3, the outer flange portion 13 can be engaged with a pair of transfer rails R for transferring the barrel 10.

The barrel 10 is provided, on the internal diameter thereof on the nozzle portion 12 side, with an even-diameter portion 14 having no protrusions thereon, and the barrel 10 is provided, on the internal diameter thereof on the outer flange portion 13 side, with a female thread portion 15 which has a greater diameter than that of the even-diameter portion 14. The barrel 10 is provided on an inner peripheral surface thereof with a stepped portion 16 which lies in a plane perpendicular to a common axis of the connecting even-diameter portion 14 and the female thread portion 15. The stepped portion 16 is provided with a plurality of air evacuation grooves 17 at predetermined intervals (intervals of 90 degrees in the illustrated embodiment) so that each air evacuation groove 17 communicatively connects with the inner peripheral surface of the even-diameter portion 14 (see Figure 2).

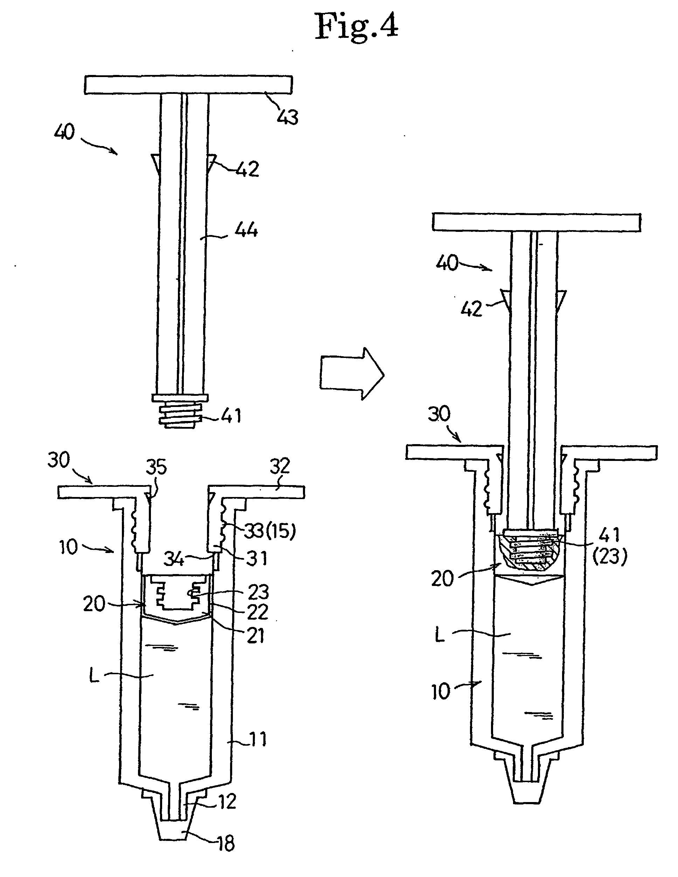

The stopper 20 is a film-coated stopper consisting of a resilient core 21 and a film 22 which is inert with respect to the pre-filled liquid drug L. The film 22 is made of, e.g., a fluorocarbon resin or an ultrahigh molecular weight polyethlene. Specifically, the film 22 is desirably made of a fluorocarbon resin. The film 22 covers at least the portion of the outer surface of the resilient core 21 which is in contact with at least one of the pre-filled liquid drug L and the even-diameter portion 14. The resilient core 21 is provided along the axis thereof with a threaded screw hole 23 in which a plunger 40 (see Figures 4 through 6) is screwed into. The outside diameter D of the stopper 20 in a free state is slightly greater than the inside diameter d1 of the even-diameter portion 14 of the barrel 10 in a free state (see Figure 2). Therefore, in a state where the stopper 20 is simply mounted on the stepped portion 16, the inside and outside of the barrel 10 (even-diameter portion 14) are communicatively connected with each other via the air evacuation grooves 17. The air evacuation grooves 17 can be modified as beveled grooves to gradually increase the diameter of the barrel 10 around the outer flange portion 13 in the direction toward the outer flange portion 13 so that the stopper 20 can easily be inserted into the barrel 10, or edges of the air evacuation grooves 17 can be beveled so as to prevent the stopper 20 from being scratched by the edges of the air evacuation grooves 17.

The flanged fixing member 30 is provided with a cylindrical portion 31 and a finger flange 32 extending radially outwards from the outer end of the cylindrical portion 31. The cylindrical portion 31 is provided on an outer peripheral surface thereof with a male thread portion 33 which is in mesh with the female thread portion 15 of the barrel 10. The flanged fixing member 30 is made of synthetic resin, e.g., a cycloolefin resin which is the same material as that of the barrel 10, or a synthetic resin whose degree of resiliency is less than that of the barrel 10 (i.e., a synthetic resin softer than the material of barrel 10). The inside diameter d2 of the cylindrical portion 31 of the flanged fixing member 30 is sufficiently smaller than the outside diameter D of the stopper 20, and the inner end face of the cylindrical portion 31 serves as a stopper-pull-out prevention portion (stopper-pull-out prevention end face) 34.

The flanged fixing member 30 is provided on an inner peripheral surface of the cylindrical portion 31 with an annular non-pull-out notch 35. The plunger 40 (see Figures 4 through 6) which has a threaded stem portion 41 screwed into the threaded screw hole 23, is provided with reuse-prevention projections 42. The reuse-prevention projections 42 are engaged with the non-pull-out notch 35 when the plunger 40 is inserted into the barrel 10 up to a position therein whereby the liquid drug L is totally discharged. The plunger 40 is provided, at the opposite end thereof from the threaded stem portion 41, with a thumb rest 43. The maximum diameter of a shaft portion 44 of the plunger 40 is much smaller than the inside diameter d2 of the cylindrical portion 31 of the flanged fixing member 30.

The barrel 10, the stopper 20 and the flanged fixing member 30, which have been described above, are assembled in a manner shown in Figure 1. The barrel 10, the stopper 20 and the flanged fixing member 30 are sterilized in advance. A cap 18 which is also sterilized is put on the nozzle portion 12. A predetermined amount of the liquid drug L is put into the even-diameter portion 14 of the barrel 10 before the stopper 20 is mounted on the stepped portion 16, and air is removed from the barrel 10 in a vacuum. As described above, in a state where the stopper 20 is simply mounted on the stepped portion 16, the air in the barrel 10 can be discharged therefrom via the air evacuation grooves 17. Subsequently, after the air in the barrel 10 is discharged sufficiently, the stopper 20 is press-fitted into the even-diameter portion 14 of the barrel 10 until the stopper comes into contact with the liquid drug L.

Subsequently, the flanged fixing member 30 is screwed into the open end of the barrel 10 into which the stopper 20 has been press-fitted in the aforementioned manner. Namely, the male thread portion 33 of the flanged fixing member 30 is fully screwed into the female thread portion 15 of the barrel 10. In this state, the stopper-pull-out prevention portion 34, which is provided at the inner end of the cylindrical portion 31, prevents the stopper 20 from being pulled out of the barrel 10.

The barrel 10 which has been filled with the liquid drug L and to which the stopper 20 and the flanged fixing member 30 have been fixed in the above described manner is shipped to a medical facility as a product provided separate from the plunger 40 as shown in the left drawing in Figure 4, or as a product after the threaded stem portion 41 of the plunger 40 has been screwed into the threaded screw hole 23 of the stopper 20 as shown in the right drawing in Figure 4.

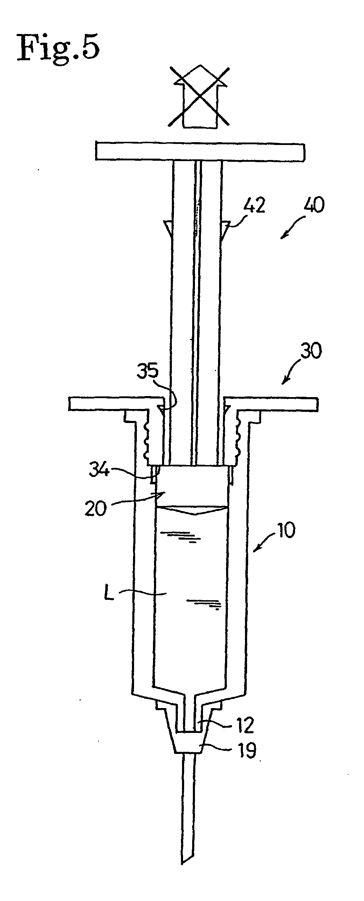

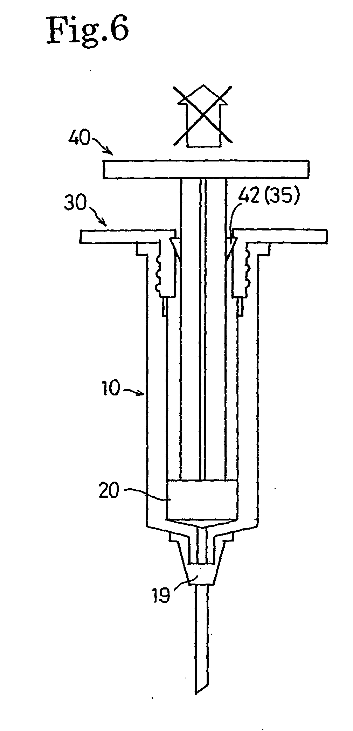

In the medical facility, when the liquid drug L needs to be given to a patient, the cap 18 is removed first, and subsequently an injection needle 19 is put on the nozzle portion 12 as shown in Figures 5 and 6. Thereafter, the injection needle 19 is inserted into the skin of a patient or a high-pressure medicinal liquid which is to be mixed with the liquid drug L. At this stage, if a high pressure is exerted on the liquid drug L in the barrel 10 via the injection needle 19 such as to force the stopper 20 out of the barrel 10, the stopper-pull-out prevention portion 34 of the cylindrical portion 31 of the flanged fixing member 30 that is screwed into the open end of the barrel 10 prevents the stopper 20 from being forced out of the barrel 10 (see Figure 5). Since the flanged fixing member 30 is screwed into the barrel 10, the force which the flanged fixing member 30 can withstand is very strong. Accordingly, the stopper 20 cannot be pulled out of the barrel 10, so that the liquid drug L is never spilled out of the barrel 10 accidentally.

Pushing the plunger 40, having been screwed into the stopper 20, into the barrel 10 to discharge the liquid drug L out of the barrel 10 causes the reuse-prevention projections 42 to be engaged in the non-pull-out notch 35 of the flanged fixing member 30 (see Figure 6). Therefore, the plunger 40 cannot be pulled out of the barrel 10. This prevents the syringe assembly from being reused. The flanged fixing member 30 can be bonded to the barrel 10 after screwed into the barrel 10 to further ensure the above described stopper-pull-out prevention function and reuse prevention function. If the flanged fixing member 30 is made of a relatively soft material, the reuse-prevention projections 42 of the plunger 40 can be engaged in the non-pull-out notch 35 with a smaller resistance. However, if the material of the flanged fixing member 30 is excessively soft, the reuse-prevention projections 42 engaged in the non-pull-out notch 35 may disengage therefrom accidentally. Accordingly, it is desirable that the material of the flanged fixing member 30 be selected in consideration of the ability of the flanged fixing member 30 to keep the plunger 40 inserted in the flanged fixing member 30 and the ability of the flanged fixing member 30 to prevent the plunger 40 from being disengaged from the flanged fixing member 30.

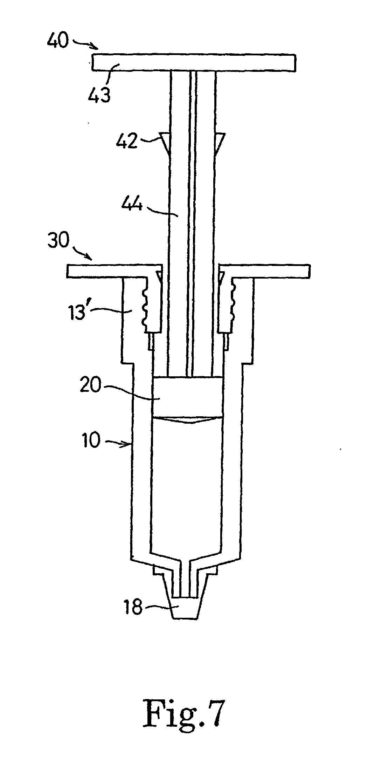

Figure 7 shows another example of the barrel 10, the shape of which being different from the shape of the barrel 10 shown in Figure 1. In this example, the barrel 10 is provided around the open end thereof with a thick wall portion 13'. This example of the barrel 10 can be regarded as the same as the barrel 10 shown in Figure 1 if modified so that the outer flange portion 13 shown in Figure 1 is elongated in the axial direction of the barrel 10. According to the barrel 10 shown in Figure 7, deterioration of the strength of the barrel 10 due to the formation of the female thread portion 15 can be prevented.

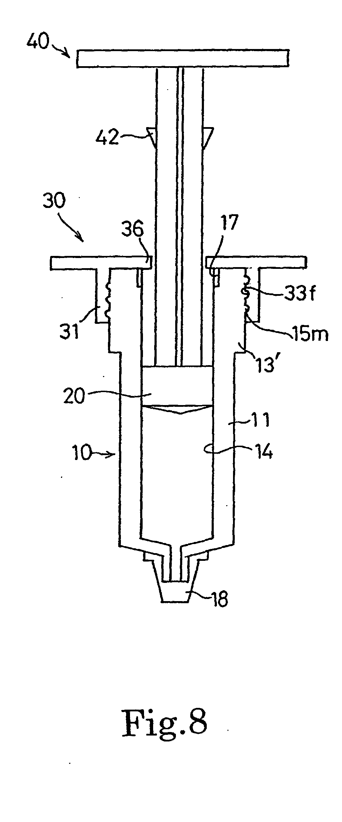

Figure 8 shows another embodiment of the pre-filled syringe assembly in which the thick wall portion 13' of the barrel 10 is provided on an outer peripheral surface thereof with a male thread portion 15m while the flanged fixing member 30 is provided on an inner peripheral surface of the cylindrical portion 31 with a female thread portion 33f which is in mesh with the male thread portion 15m to fix the flanged fixing member 30 to the barrel 10 via engagement of the thread. In this embodiment of the pre-filled syringe assembly, the flanged fixing member 30 is provided with an inwardly extending flange 36 formed at the same axial position as that of the finger flange 32. The inwardly extending flange 36 prevents the stopper 20 from being pulled out before discharge of the liquid drug L, and also prevents the plunger 40 from pulling out of the barrel 10 after discharge of the liquid drug L. The even-diameter portion 14 having no protrusions thereon is formed in the inner circumferential surface of the barrel 10, and the plurality of air evacuation grooves 17 are formed at the open end of the even-diameter portion 14. This embodiment has the advantage of preventing the stopper 20 from being pulled out of the barrel before usage and preventing the plunger 40 from being pulled out of the barrel 10 after usage due to the inwardly extending flange 36 being formed on the flanged fixing member 30.

Although the reuse-prevention projections 42 are formed on the plunger 40 while the non-pull-out notch 35, in which the reuse-prevention projections 42 can be engaged, is formed on the flanged fixing member 30 in the above illustrated embodiment, the reuse-prevention projections 42 can be formed on flanged fixing member 30 while the non-pull-out notch 35 can be formed on plunger 40.

### INDUSTRIAL APPLICATION OF THE INVENTION

According to the present invention, a pre-filled syringe assembly having the capability of preventing the stopper from being pulled out of the barrel with a sufficient strength with no possibility of scratching the stopper when the stopper is inserted into the barrel is achieved since the stopper is prevented from being pulled out of the barrel by a flanged fixing member which is screwed into the open end of the cylindrical barrel. In addition, since the annular non-pull-out notch in which the reuse-prevention projections are engageable can be formed on the flanged fixing member, the syringe assembly can be prevented from being reused.

## Claims

1. A pre-filled syringe assembly comprising:
a cylindrical barrel having one of a cap and an injection needle provided on one end of said cylindrical barrel, the other end of said cylindrical barrel being formed as an open end;
a flanged fixing member having a cylindrical portion and a finger flange, said cylindrical portion being formed to correspond to said open end of said cylindrical barrel, and said finger flange extending radially outwards from said cylindrical portion;
two threaded portions formed on said cylindrical barrel around said open end thereof and said cylindrical portion of said flanged fixing member, respectively, to fix said flanged fixing member to said cylindrical barrel; and
a stopper-pull-out prevention portion formed on said flanged fixing member to have a diameter smaller than each of an inside diameter of said cylindrical barrel and an outside diameter of a stopper fitted into said cylindrical barrel.

2. The pre-filled syringe assembly according to claim 1, further comprising:
a plunger screwed into a core of said stopper;
a reuse-prevention projection formed on one of said plunger and said flanged fixing member; and
a non-pull-out recess formed on the other of said plunger and said flanged fixing member, said reuse-prevention projection being engaged in said non-pull-out recess when said plunger is inserted into said barrel up to a position therein whereby a liquid drug pre-filled in said barrel is totally discharged.

3. The pre-filled syringe assembly according to claim 1 or 2, wherein said barrel is provided, on an inner peripheral surface thereof on the open end side of said barrel, with an air evacuation groove which forms a gap between said barrel and said stopper when said stopper is positioned at the open end side of said barrel.

4. The pre-filled syringe assembly according to any one of claims 1 through 3, wherein said two thread portions, which are formed on said cylindrical barrel around said open end thereof and said cylindrical portion of said flanged fixing member, comprise a female thread portion and a male thread portion, respectively.

5. The pre-filled syringe assembly according to claim 4, wherein said stopper-pull-out prevention portion of said flanged fixing member is formed by an inner end of said cylindrical portion.

6. The pre-filled syringe assembly according to claim 2, wherein said two thread portions, which are formed on said cylindrical barrel around said open end thereof and said cylindrical portion of said flanged fixing member, comprise a female thread portion and a male thread portion, respectively, and
wherein said reuse-prevention projection or said non-pull-out recess which is formed on said flanged fixing member is formed on an inner peripheral surface of said cylindrical portion of said flanged fixing member.

7. The pre-filled syringe assembly according to any one of claims 1 through 3, wherein said two thread portions, which are formed on said cylindrical barrel around said open end thereof and said cylindrical portion of said flanged fixing member, are a male thread portion and a female thread portion, respectively.

8. The pre-filled syringe assembly according to claim 7, wherein said stopper-pull-out prevention portion of said flanged fixing member is formed by an inwardly extending flange formed at substantially the same axial position as that of said finger flange of said flanged fixing member.

9. The pre-filled syringe assembly according to claim 2, wherein said two thread portions, which are formed on said cylindrical barrel around said open end thereof and said cylindrical portion of said flanged fixing member, are a male thread portion and a female thread portion, respectively, and
wherein said stopper-pull-out prevention portion and said non-pull-out recess of said flanged fixing member are formed by an inwardly extending flange formed at substantially the same axial position as that of said finger flange of said flanged fixing member.

10. The pre-filled syringe assembly according to claim any one of claims 1 through 9, wherein said barrel is made of a cycloolefin resin.

11. The pre-filled syringe assembly according to any one of claims 1 through 10, wherein said stopper is coated with a film which is inert with respect to a liquid drug which is pre-filled in said barrel, said film covering at least a portion of said stopper which is in contact with at least one of said pre-filled liquid drug and said barrel.
